# EUROPEAN PATENT SPECIFICATION

(11) **EP 4 034 864 B1**
(45) Date of publication and mention of the grant of the patent: **09.10.2024**
(21) Application number: 20758145.5
(22) Date of filing: 14.08.2020
(51) Int. Cl.: G01N 21/31, G01N 21/47, G01N 21/51, G01J 3/02, G01J 3/42, G01N 33/487, A61B 5/00, A47G 19/22, A61B 10/00, A61C 17/22, A61B 5/1455, A61B 5/02, A46B 15/00, A61C 19/04, G01N 21/01

(54) **PROVIDING AN INDICATION OF A PERSON'S GUM HEALTH**
BEREITSTELLUNG EINES HINWEISES AUF DIE ZAHNFLEISCHGESUNDHEIT EINER PERSON
FOURNITURE D'UNE INDICATION DE LA SANTÉ DES GENCIVES D'UNE PERSONNE

(30) Priority: 15.08.2019 WO PCT/EP2019/071926; 14.02.2020 EP 20157431
(43) Date of publication of application: 03.08.2022
(73) Proprietor: Koninklijke Philips N.V., 5656 AG Eindhoven (NL)
(72) Inventor: RONDA, Cornelis, Reinder, 5656 AE Eindhoven (NL); VERMEULEN, Olaf, Thomas, Johan, Antonie, 5656 AE Eindhoven (NL); SCHEFFERS, Lucas, Petrus, Henricus, 5656 AE Eindhoven (NL)
(74) Representative: Philips Intellectual Property & Standards
(86) International application number: PCT/EP2020/072825
(87) International publication number: WO 2021/028557

(56) References cited:
- EP-A1- 3 441 764
- EP-A1- 3 611 494
- WO-A1-2015/069704
- WO-A1-2015/116854
- WO-A1-2016/095202
- WO-A1-2019/115303
- WO-A1-84/03143
- US-A1- 2002 127 143
- US-A1- 2011 050 431
- PRASANTH CHANDRA SEKHAR ET AL: "Discrimination of periodontal diseases using diffuse reflectance spectral intensity ratios", JOURNAL OF BIOMEDICAL OPTICS, 5 March 2012 (2012-03-05), United States, pages 027001 - 1, XP055180954, Retrieved from the Internet <URL:http://dx.doi.org/10.1117/1.JBO.17.2.027001> [retrieved on 20150402], DOI: 10.1117/1.JBO.17.2.027001

## Description

### FIELD OF THE INVENTION

The invention relates to a system configured to provide an indication of a person's gum health.

The invention also relates to a method of providing an indication of a person's gum health. In this respect, the invention also relates to a computer program product comprising code to cause a processor, when the code is executed on the processor, to cause the system as mentioned to execute the method as mentioned. Further, in this respect, the invention also relates to a remote device having a processor, comprising the computer program product as mentioned.

### BACKGROUND OF THE INVENTION

WO 84/03143 A1 discloses a probe for insertion into a periodontal pocket, for measuring a depth. It uses analysis of reflected light to determine a depth of saliva in the pocket and hence a pocket depth.

US 2011/050431 A1 discloses a beverage container incorporating a sensor, for example for determining an amount of glucose and carbohydrates in the beverage.

EP 3 441 764 A1 discloses a system for analyzing a blood content in saliva in order to determine a stage of periodontal disease.

In the field of oral healthcare, hygiene and health of teeth and gums are of utmost importance. In order to ensure an optimal level of hygiene and health of teeth and gums, regular inspection of various oral conditions is needed. One of the common problems that may occur and that may go unnoticed if no specific inspection is done is gingivitis.

Gingivitis is inflammation of the gums, characterized by swollen gums, oedema and redness. The primary cause of gingivitis is plaque build-up, mostly in the gingival sulcus (pockets). Gingivitis most often occurs in hard to reach areas, such as interproximal areas, i.e. areas between adjacent teeth, and around the posterior teeth, which are difficult to clean properly.

Gingivitis can be reversed by improved oral hygiene, especially in an early stage of the inflammation. As gingivitis can propagate to irreversible periodontitis, it is important to maintain a high level of oral health and to detect gingivitis as soon as possible.

It is known to visually diagnose gingivitis by assessing reddening and swelling of the gingiva. For example, it is known to use a non-contact gingivitis index. However, this way of doing has limited sensitivity and is highly dependent on the color rendering index of the light source that is used in the process, e.g. modern phosphor converted LEDs can have a low CRI (Color Rendering Index) resulting in poor visual judgements. In general, self-diagnosis of gingivitis is difficult, all the more since gingivitis often occurs at the back of the mouth, as mentioned earlier.

Another known method for assessing whether a person suffers from gingivitis involves the use of a probe for investigating tissue areas in a person's mouth and enabling detection of gingivitis on the basis of DRS (Diffuse Reflective Spectroscopy). Applying DRS techniques offers the possibility to determine increased total hemoglobin concentration and decreased blood oxygenation related to gingivitis. Although DRS enables a sensitive method of detecting moderate gingivitis, it has some disadvantages, which reside in the facts that accurate positioning of the probe is required, that it is sensitive to movements, and that the measurement can be corrupted when blood is pushed away from the tissue under investigation when direct contact between the probe and the tissue takes place at too high force.

### SUMMARY OF THE INVENTION

It is an object of the invention to fulfill the need for easy and reliable detection of moderate gingivitis. To put it more generally, it is an object of the invention to fulfill the need for easy and reliable assessment of gum health. Preferably, the detection should be of such nature that it can easily be combined with normal/daily oral hygiene routines such as brushing and flossing.

The invention is defined by the independent claims. The dependent claims define advantageous embodiments,

It is estimated that 50% to 70% of adults have gingivitis, but until the inflammation has become so severe that their gums bleed heavily during brushing, they are unaware of it. In the early stages of gingivitis, the gums bleed as well during brushing, but the amount of blood loss is so low that it is not or hardly noticeable with the naked eye in saliva or a fluid-saliva mixture such as a toothpaste-saliva mixture. When colored toothpaste is used, noticing blood loss becomes even more difficult, especially when the toothpaste is red. The invention is based on an insight that it is possible to diagnose gingivitis on the basis of measurements aimed at determining whether or not blood is present in saliva or a fluid-saliva mixture, wherein the saliva or fluid-saliva mixture may be obtained during an action in the person's mouth involving a gum agitation effect, or after such an action has taken place.

In accordance with the invention, such an action is an oral care action such as brushing or flossing. In the context of the invention, a sufficiently sensitive method has been developed by means of which it is possible to trace even very small quantities of blood in saliva or a fluid-saliva mixture, so that gingivitis may be diagnosed in an early stage.

When the invention is put to practice, an optical analysis of saliva or a fluid-saliva mixture is performed in order to check whether the saliva or fluid-saliva mixture contains blood. In particular, light received from a representative volume of fluid containing saliva is detected and analyzed, which light is obtained by emitting light to the volume of fluid and receiving the light back from the volume of fluid. The analysis is done in a particular way and comprises the steps as defined in the foregoing. Advantageously, as mentioned, investigating a representative volume of fluid is done during or after an oral care action is performed in the person's mouth.

The invention relies on determination of one or more measurement values of light received by the light-receiving unit for no more than a single wavelength of the light. According to the invention, the single wavelength of the light is a wavelength related to relatively high light absorption by a constituent of blood, wherein it is noted that the constituent of blood as mentioned may particularly be hemoglobin.

The invention is based on the insight that the measurement configuration as determined by the positioning of the light-emitting unit and the light-receiving unit with respect to each other and the volume of fluid between the respective units can be chosen such that it is possible to regard the volume of fluid as a semi-infinite layer, which implies that out of the three factors normally determining the features of the light received by the light-receiving unit, i.e. reflection, absorption and transmission, transmission may be regarded to be zero. Further, a presence of blood in a volume of fluid mainly influences the absorption of the light, especially when a wavelength of the light is chosen to be a wavelength related to relatively high light absorption by a constituent of blood, as is the case according to the invention. When blood is present, absorption of the light is increased and reflection of the light is decreased. Thus, it is expected that the intensity of reflected light can be taken as a measure of the extent to which the light is absorbed by the volume of fluid, as the absorption of the light by the volume of fluid becomes the only factor changing reflection of the light, and thereby a measure of an extent to which blood is present in the volume of fluid.

The results of tests performed in the context of the invention have confirmed that in a practical measurement configuration, it is justified to regard a volume of fluid such as a volume of a toothpaste-saliva mixture as a semi-infinite layer, indeed. Hence, the results of those tests indicate that performing light reflection measurements enable obtaining a useful indication about the absence or presence of blood in a volume of fluid containing a person's saliva, as will be explained further in the detailed description of embodiments with reference to the figures.

With reference to the foregoing general explanation of the invention, it is noted that it is advantageous if the light-receiving unit is configured to receive reflected light back from the volume of fluid, so that the measurements at the single wavelength can be performed in a proper manner and useful measurement results may be expected.

In the context of the invention, it may be practical if the at least one algorithm is designed to determine measurement values real-time during an action in the person's mouth involving a gum agitation effect, and to monitor a development of the measurement values over time and assess whether a deviation from a gradual course of the development of the measurement values occurs in the development. As long as blood does not appear from the gums and/or as long as only fluid dilution effects such as dilution of toothpaste take place, a gradual course of development of the measurement values may be expected, i.e. the development of the measurement values may be expected to be of a more or less steady/constant character. However, a deviation from a gradual course of the development of the measurement values may indicate that a release of blood from the gums has taken place. Thus, by determining measurement values real-time and assessing whether a "jump" occurs, it can be found that blood is released from a spot in the mouth. The more times this happens throughout a measurement period, the worse the gum health appears to be. In respect of the term "action involving a gum agitation effect", it is noted that this should be understood so as to cover any action that might cause some form of mechanical impact on the gums. Hence, such an action may involve direct contact to the gums, but may also involve fluid pressure on the gums, for example.

For example, the at least one algorithm may be designed to determine the measurement values at successive discrete moments in time, and to involve determining differences between successive measurement values in assessing whether a deviation from a gradual course of the development of the measurement values occurs in the development. In such a case, the at least one algorithm may particularly be designed to involve checking whether each of differences from at least a predetermined number of successive differences is larger than each of differences from at least a predetermined number of preceding differences by at least a predefined minimum percentage in assessing whether a deviation from a gradual course of the development of the measurement values occurs in the development. In any case, the at least one algorithm may be designed to determine that the measurement values are indicative of the presence of blood in a volume of fluid containing saliva when a deviation from a gradual course of the development of the measurement values is found in the development.

It is known that the hemoglobin of blood has a light absorption spectrum in which absorption peaks can clearly be distinguished at wavelength values in a range of about 400 to 440 nm. For wavelength values of about 440 nm or higher, the absorption of hemoglobin is significantly lower. This is true for both oxyhemoglobin, i.e. hemoglobin with bound oxygen, and deoxygenated hemoglobin, i.e. hemoglobin without bound oxygen. Hence, for the purpose of determining whether or not a volume of fluid contains blood, it is advantageous if an optical measurement result is considered at a wavelength in the range of about 400 to 440 nm. In view thereof, a practical example of the single wavelength of the light is a wavelength in the said range of 400 to 440 nm. For the sake of clarity and correct interpretation of the present text including the claims, it is noted that where a single wavelength is mentioned, or where a specific wavelength value is mentioned in relation to a device such as a light source or a filter, this is to be understood for its practical meaning rather than its theoretical meaning, taking into account that in fact a tolerance window is applicable.

In a general sense, the at least one measurement value of light received by the light-receiving unit may be regarded as being representative of information that is related to a person's gum health in some way, and as being suitable to be used for the purpose of determining output representative of the person's gum health. In conformity with what has already been suggested in the foregoing, it is possible for the determination of the at least one measurement value to be followed by a further step that involves determination whether or not the at least one measurement value is indicative of the presence of blood in a volume of fluid containing saliva. Such a further step may be performed in any appropriate way, and may involve, for example, comparing the at least one measurement value to at least one reference value, or keeping track of a development of measurement values over time in order to see whether deviations in a gradual course of the development can be found, as explained in the foregoing. For the purpose of informing a user, it may be advantageous if a warning signal is emitted in case an indication of the presence of blood in a volume of fluid under investigation is found. To that end, the system according to the invention may be equipped with any suitable type of warning mechanism as will further be explained in the following.

The analysis unit of the system according to the invention is configured to perform at least the determination of the at least one measurement value. Hence, output of the analysis unit may come in the form of the at least one measurement value. It is also possible that the analysis unit is configured to perform the further step, in which case output of the analysis unit may come in a form that does not require further interpretation but is a direct indication as to whether or not blood is present in a volume of fluid and/or whether or not oral health issues may be assumed. In any case, for the purpose of indicating the likelihood that the person suffers from gingivitis or another condition affecting gum health, it is sufficient to detect whether or not blood is present in the person's saliva after the person's gums have been agitated in some way, or during such a process.

A notable advantage of the invention resides in the fact that there is no need for performing complex actions or using sophisticated and expensive equipment for performing the analysis of a volume of fluid containing saliva. In the first place, it is possible to put the invention to practice in such a way that all that is needed for obtaining a volume of fluid that is suitable for investigation is performing a normal oral care action. Within the scope defined by the appended claims, the volume of fluid can be obtained in various ways, ranging from having an area on an oral care appliance that is particularly shaped for this purpose to having a person spit out some saliva. Further, commonly available light sources such as LEDs may be used as light-emitting units, possibly in combination with optical filters, and commonly available light detectors such as photodiodes may be used as light-receiving units, wherein there is no need for an expensive spectrometer. The analysis unit may be provided in the form of a preprogrammed microprocessor or a general microprocessor in combination with a suitable app or the like for controlling operation of the microprocessor, to mention two practical examples.

As explained earlier, a notable aspect of the invention is the fact that processing at least one measurement value relating to no more than a single wavelength is considered to be sufficient for determining whether or not a volume of fluid contains blood (actually, the hemoglobin of the blood or possibly another constituent of the blood). In view thereof, two of the possibilities existing in respect of the system according to the invention are the following: the light-emitting unit is configured to emit light at only the single wavelength, and may, for example, comprise a suitable LED to that end, and the light-receiving unit comprises a bandpass filter/light detector combination including a bandpass filter that is configured to only allow light at the single wavelength or a narrow wavelength band to pass.

The possibilities as mentioned are based on the insight that if it is sufficient to analyze at least one measurement value related to only a single wavelength, it may be sufficient to generate only such at least one measurement value and no more. An advantage of generating only the one or more measurement values needed for the analysis is that cheap and commonly available components may be applied.

As suggested in the foregoing, in a practical embodiment, the system according to the invention may be equipped with a warning mechanism configured to emit a signal in case the at least one measurement value is analyzed and found to be indicative of the presence of blood in a volume of fluid. The signal may be a visible signal and/or an audible signal, for example. In a general sense, the system according to the invention may be equipped with an output interface configured to communicate the information about gum health in a person's mouth, as determined and output by the analysis unit, to a user of the output interface, who may be the person herself/himself and/or another person such as a dentist. Outputting the information may be done through an app on the user's phone, for example, or on a suitable display. The output interface is adapted to generate output in a humanly processable form, i.e. in a form that can be detected by at least one of the human senses and processed in the human brain so as to make a human aware of the output of the output interface. Practical examples of output in a humanly processable form include output as can be detected by human vision, output as can be detected by human hearing and tactile output. In respect of the latter example, it is noted that when the invention involves use of an oral care appliance such as a power toothbrush, tactile output may be provided by varying the frequency and/or the amplitude of the motion of a functional unit (head) thereof. The analysis unit may be configured to transmit an information signal as a wireless signal, wherein there is no need for the analysis unit and the output interface to be physically connected in any way. The internet may be used for conveying the information signal to any person to whom the information generated by the analysis unit may be of interest. The results of successive analysis actions may be saved and may be subjected to further analysis so as to perform an assessment of trends over time.

Various known devices and objects may be adjusted so as to be capable of putting to the invention to practice and to thereby provide an indication of a person's gum health. For example, an embodiment of the system according to the invention is feasible in which the system comprises a remote device having a processor, wherein the analysis unit comprises the processor of the remote device, and wherein the at least one algorithm to be executed by the analysis unit is defined by an app installed on the remote device. The remote device may also comprise a light module and an image chip, in which case it may be advantageous if the light-emitting unit comprises the light module of the remote device and the light-receiving unit comprises the image chip of the remote device. Examples of a remote device include a mobile/portable device such as a smartphone, a smartwatch, a tablet or a laptop, and also a non-portable computer. Other configurations are possible, such as a configuration in which an app by means of which the at least one algorithm is defined is not installed on a mobile/portable device or a non-portable computer but on a remote server that is accessible by a mobile/portable device or a non-portable computer, or a configuration in which the analysis unit is arranged outside of a mobile/portable device or a non-portable computer, in which case the mobile/portable device or the non-portable computer can be provided with an app that is designed to enable measurement data to be transmitted to the analysis unit.

According to another possibility of putting the invention to practice, the system comprises a power toothbrush or power flosser configured to perform an oral care action that involves a gum agitation effect, wherein at least the light-emitting unit and the light-receiving unit are incorporated in the oral care appliance. It may be so that the oral care appliance has a handle portion and a head portion, in which case the light-emitting unit and the light-receiving unit may be arranged at the position of the head portion.

The disclosure also relates to a handle portion of an oral care appliance, the oral care appliance being configured to perform an oral care action that involves a gum agitation effect and further having a head portion, and the handle portion being configured to be used with the head portion and to be used in the system disclosed in the foregoing, wherein the handle portion accommodates at least the analysis unit of the system.

As explained in the foregoing, it may be very convenient to a user if the process of collecting input for assessing a person's gum health can take place during an oral care action that he/she is used to, in which case the process practically goes unnoticed. In the above-mentioned case of the system according to the invention comprising an oral care appliance, it may be so that the oral care appliance is configured to be used in both a process of performing an oral care action which may lead to the release of blood from the gums in case of reduced or poor gum health, and a process of determining whether or not blood is present in the saliva. It is possible to obtain a real-time gum health indication during use of the oral care appliance, and also to obtain a range of localized indications in respect of various locations in a person's mouth if measures are taken for keeping track of a position of (a part of) the oral care appliance with respect to the person's mouth. The analysis unit may be provided outside of the oral care appliance, in which case it is advantageous if the oral care appliance is equipped with at least one unit that is designed to communicate with the analysis unit in a wireless fashion and/or another appropriate fashion.

Within the scope defined by the appended claims, it may be advantageous for an oral care appliance that is designed for use in the system according to the invention to comprise an area for accommodating a volume of fluid containing a person's saliva realized as a recess in the oral care appliance. This provides a possibility of collecting and evaluating the volume of fluid containing the person's saliva in the person's mouth, which is an optimal way of obtaining an indication whether or not a person may suffer from reduced or poor gum health during an oral care routine performed on a regular basis.

In an example which does not fall within the scope of the invention, it is also possible to apply a container of which at least a bottom is transparent to light, particularly light at the single wavelength, in which case the volume of fluid containing a person's saliva can be obtained by letting the person under investigation spit in the container, and light reflection can be measured at the single wavelength.

The above-described and other aspects of the invention will be apparent from and elucidated with reference to the following detailed description of aspects of the theoretical background of the invention and a number of practical ways of putting the invention to practice.

### BRIEF DESCRIPTION OF THE DRAWINGS

The invention will now be explained in greater detail with reference to the figures, in which equal or similar parts are indicated by the same reference signs, and in which:
Fig. 1 shows absorption spectra of hemoglobin;
Fig. 2 illustrates the basic setup of a test arrangement for detecting a presence of blood in a toothpaste-saliva mixture;
Fig. 3 is a graph of measurement values of reflected light plotted against concentration of blood in a toothpaste-saliva mixture;
Fig. 4 is graph of normalized measurement values of reflected light plotted against concentration of blood in a toothpaste-saliva mixture, for three different concentrations of toothpaste;
Fig. 5 is an example of a diffuse reflective probe;
Fig. 6 is an example of a reflection measurement realized in a drinking glass; and
Figs. 7 and 8 illustrate how the invention may be realized in an oral care appliance.

### DETAILED DESCRIPTION OF THE EMBODIMENTS

The invention, defined by the appended claims, provides a practical way of providing an indication of a person's gum health. According to the invention, the presence of blood traces in a volume of fluid containing a person's saliva is evaluated by performing optical measurements and analyzing the results of the measurements, wherein an appropriate choice is made when it comes to a single wavelength at which the results are considered in the process.

The invention is particularly aimed at providing a way of detecting blood traces in saliva or a toothpaste-saliva mixture for the purpose of obtaining an indication that the person whose saliva is under investigation may suffer from (early stage) gingivitis or from another condition affecting gum health. In view thereof, the invention provides a reliable method for detecting low hemoglobin concentrations, by using optical detection based on the known absorption peaks in the hemoglobin absorption spectrum. As is generally known, hemoglobin is an important constituent of blood.

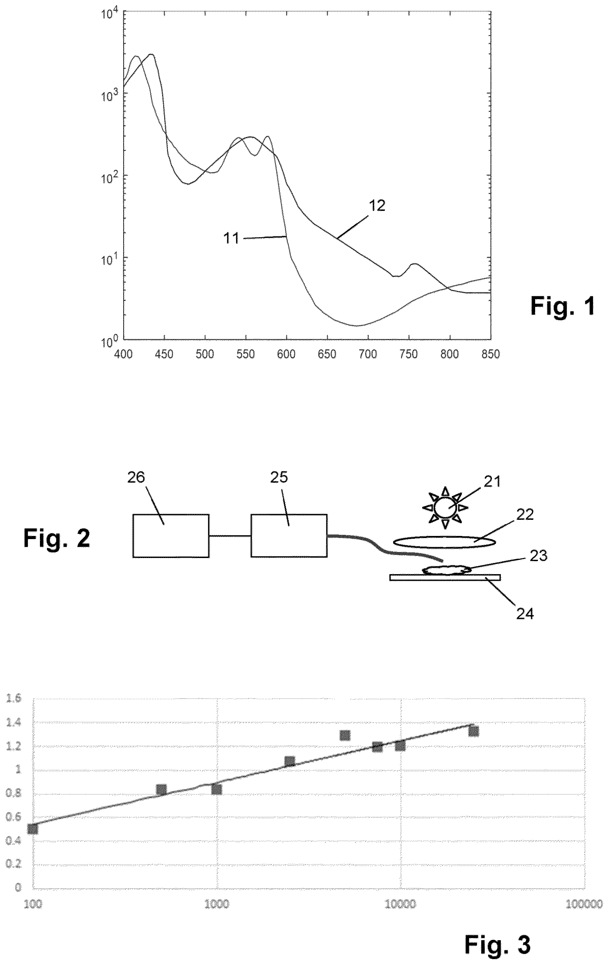

Fig. 1 shows two absorption spectra of hemoglobin, wherein the spectrum that is indicated by reference numeral 11 is related to oxyhemoglobin, i.e. hemoglobin with bound oxygen, and wherein the spectrum that is indicated by reference numeral 12 is related to deoxygenated hemoglobin, i.e. hemoglobin without bound oxygen. The wavelength of light expressed in nm is at the x axis of the spectra, and the absorption coefficient expressed in cm⁻¹ is at the y axis of the spectra. It appears from the figure that there are some specific, highly absorbing wavelength ranges. Especially the blue wavelength range of 410 to 420 nm involves relatively high values of the absorption coefficient of the hemoglobin. The broader range of high absorption is 400 to 440 nm.

In the context of the invention, it is sufficient to check whether or not blood is present in a volume of fluid containing saliva, wherein there is no need for determining the exact value of the concentration of the blood in the volume of fluid. The invention involves a method of assessing optical behavior of a volume of fluid containing saliva, which volume of fluid may be obtained during or after an action in a person's mouth involving a gum agitation effect, an oral care action selected from brushing or flossing is used in the present invention to provide such action. It is known that in the wavelength range of 400 to 440 nm as mentioned, the absorption coefficient of water is relatively low. As water is the main constituent of saliva, there is no need to take the saliva factor into account. The absorption and reflection characteristics of toothpaste may be assumed to be a more or less constant factor, at least in relation to a possible presence of blood in the saliva. In the context of the invention, a notable insight is that measurements can be performed in such a way as to justify regarding the volume of fluid as a semi-infinite layer, so that transmission of light may be assumed to be zero. On the basis of the fact that the presence of blood in the saliva causes absorption of light to be high at a wavelength chosen from the above-mentioned range, wherein the absorption increases as the concentration of the blood increases, this leads to the conclusion that an indication about an extent to which blood is present can be obtained by considering reflection of light at such a wavelength.

The insight that the volume of fluid may be regarded as a semi-infinite layer has been verified by means of a test. In the test, a brush head provided with a light source and a light detector arranged between the bristle tufts, a container for containing a volume of fluid, and a mirror were used. The container was filled with a volume of toothpaste-saliva mixtures of different concentrations, and for each of those toothpaste-saliva mixtures, it was assessed whether or not the light intensity measured at the light detector would vary as a result of varying a distance between the brush head and the mirror. It was found that the light intensity remained constant regardless the distance, so that it is concluded that transmission is zero, indeed, because if such would not be the case, this would inevitably cause a variation of the light intensity. In respect of a volume of only saliva, it is to be noted that in a practical situation in which the saliva is put to motion under the influence of a moving brush head or the like, infinite layer behavior of the saliva is also applicable due to the formation of air bubbles in the saliva, as the presence of air bubbles causes light scattering effects due to the difference of refractive index between saliva and air.

To investigate the feasibility of performing an analysis of optical measurement results at only one wavelength for the purpose of determining whether or not blood may be assumed to be present in a volume of fluid under investigation, tests were performed, using a test arrangement of which the basic setup is illustrated in Fig. 2. Fig. 2 diagrammatically shows a light source 21, a collimator 22, a volume of fluid 23 to be investigated as present on a support 24, and a light detector 25 coupled to an analysis unit 26, wherein the light detector 25 is arranged so as to be in a position for receiving light from the light source 21 through reflection. Toothpaste and water were mixed in a 1:5 ratio after which small amounts of sheep blood were added to the mixture. A series of up to more than 10,000 times more toothpaste and water than blood was made to test whether the presence of blood could be found even in extremely small concentrations, comparable to the presence of a tiny drop of blood of about 1 µl in 10 ml of toothpaste-saliva mixture. The 1: 10,000 ratio can be regarded as being representative of a worst case scenario as 1 µl is an extremely small drop and a person will normally lose more blood from bleeding gums, wherein it is to be noted that a drop of normal size has a volume of about 50 µl.

Fig. 3 provides an illustration of the results of light reflection measurements for a mixture of toothpaste and saliva in the 1:5 ratio mentioned in the foregoing. The figure shows reflection values measured at a wavelength of 420 nm, plotted against blood concentration values, wherein the latter are expressed as the x value in a 1:x ratio of the blood and the mixture of toothpaste and saliva, so that an increasing x value indicates a decreasing amount of blood. The reflection values are expressed in arbitrary units. It follows from the figure that there is a relation between the reflection values and the blood concentration values, such that higher reflection values are found for lower blood concentration values, indeed, and that this relation is applicable even when 10,000 times more toothpaste and water than blood is present in a volume of fluid.

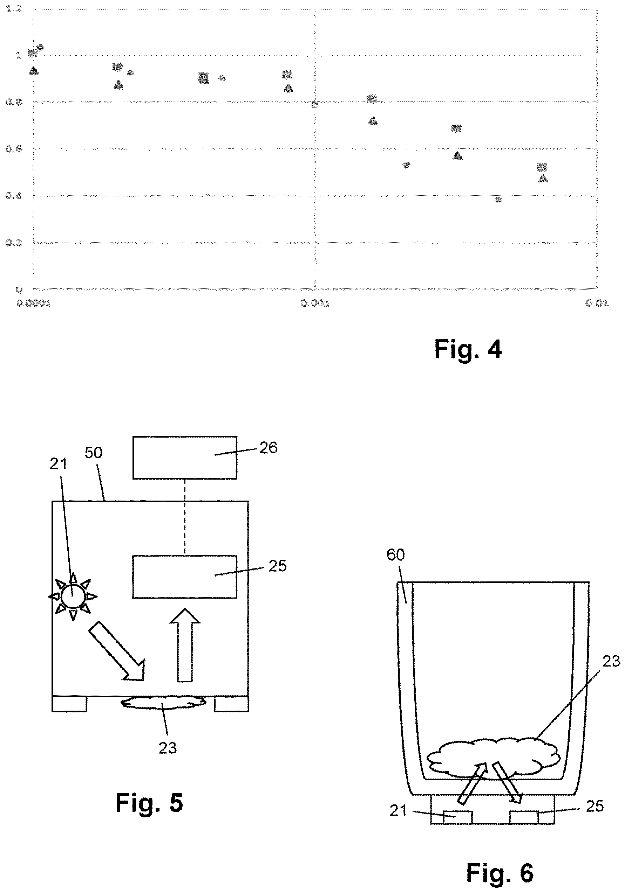

Fig. 4 provides an illustration of the results of light reflection measurements for a mixture of toothpaste and saliva in various ratios. Measurement results indicated by means of a square relate to a 1:7 ratio, measurement results indicated by means of a triangle relate to a 1:5 ratio, and measurement results indicated by means of a circle relate to a 1:3 ratio. Reflection values measured at a wavelength of 420 nm are normalized to a maximum reflection of 1, which maximum reflection is associated with the absence of blood in the toothpaste-saliva mixture. In the figure, the normalized reflection values are plotted against blood concentration values. Also this figure demonstrates that there is a relation between the reflection values and the blood concentration values, such that higher reflection values are found for lower blood concentration values, indeed.

It follows from the tests that were performed that it is possible to detect the presence of blood (hemoglobin) in a mixture of saliva and toothpaste when the optical measurement values follow from detecting and analyzing reflected light for only a single wavelength of the light. Thus, it follows from the tests that even a simple setup is sufficient for enabling detection of small amount of blood in a solution containing toothpaste. The invention provides a way of detecting much lower blood concentrations in a toothpaste-saliva mixture than can be done by the human eye, and therefore enables detection of gingivitis or other conditions affecting gum health in an early, reversible stage. The detection can be done with as little influence on normal oral care routines as possible. For example, when the invention is realized in an oral care appliance as will be explained later, it is even possible that a voluime of fluid containing saliva is collected automatically and is subjected automatically to optical analysis.

In the following, a number of examples which do not fall within the scope of the appended claims and a number of preferred ways in which the invention may be put to practice are explained. A first example relates to a diffuse reflective setup of the arrangement for obtaining measurement results suitable for use in assessing a person's gum health, which, as follows from the foregoing, involves the advantage of not requiring a separate container for receiving the volume of fluid containing saliva. This does not alter the fact that within the framework of the disclosure, it is possible to use one, as will become appararent from an explanation of a second example.

According to a first example, a diffuse reflective probe 50 as illustrated in Fig. 5 is provided, including an illumination spot coupled to a light source 21, for illumination of a volume of fluid 23 to be analyzed. Preferably, the light source 21 is modulated to reject ambient light. The diffuse reflective probe 50 further includes a light detector 25 coupled to an analysis unit 26 configured to determine whether or not blood is present in the volume of fluid 23 and to provide an indication that it is likely that a person suffers from reduced or poor gum health, or not. In the figure, paths of the emitted light and the reflected light are indicated by means of arrows. As explained in the foregoing, reflected light is detected and analyzed for only a single wavelength in order to obtain a measurement value that can be evaluated in order to see whether the measurement supports a presence of blood in the volume of fluid 23. The probe 50 may be equipped with a suitable warning mechanism for issuing a warning signal in case blood appears to be detected.

The light detector 25 can be realized in various suitable ways. For example, the light detector 25 may comprise a photodiode or a bandpass filter/detector combination. Further, the light source 21 can comprise an incandescent lamp or an LED, capable of emitting light at the single wavelength or being provided with a narrowband bandpass filter.

According to a second example existing within the framework of the disclosure, a simple drinking glass 60 is equipped with a light source 21 and a light detector 25 near a transparent bottom of the glass 60, as illustrated in Fig. 6. For the purpose of having a volume of toothpaste-saliva mixture 23 analyzed, a person spits toothpaste-saliva foam in the glass 60. Light that enters through the glass 60 from the light source 21 is reflected from the foam towards the light detector 25, as indicated by arrows in the figure. In a variation, a separate cradle for receiving and supporting a drinking glass 60 of which at least the bottom is transparent to light is used, wherein a light source 21 and a light detector 25 are integrated in the cradle.

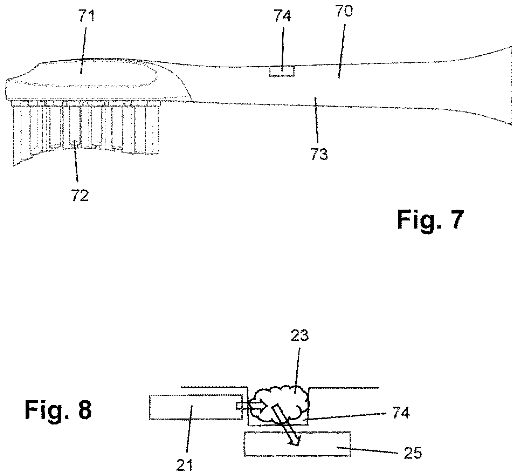

According to an embodiment existing within the framework of the invention, an oral care appliance is configured to perform one or more functions in a process of detecting blood traces in a volume of fluid containing saliva. An example of this option is illustrated in Figs. 7 and 8. In Fig. 7, a brush head portion 70 of a power toothbrush is shown. In the context of a power toothbrush, toothpaste-saliva foam will collect automatically on the brush head 71 between the bristle tufts 72 during operation of the power toothbrush. In view thereof, in accordance with the invention there may be an optical window at the position of the bristle tufts 72 in the brush head 71, and also a light source and a light detector arranged in the brush head 71, so that a light reflection measurement can be performed. The toothpaste-saliva foam will also collect automatically on the backside of the brush head 71. It may therefore also be practical if an optical window, in accordance with the invention, is provided in the backside of the brush head 71.

According to yet another possibility, within the scope defined by the claims and provided that the brush head comprises an optical window and light reflection measurement as claimed, the neck portion 73 of the brush head part 70 may be provided with a recess 74 for the purpose of collecting toothpaste-saliva foam during a brushing action. Such a recess 74 may be used for detecting a presence of blood traces through light reflection measurement, as illustrated in Fig. 8, which is the case when the light detector 25 is arranged outside of a light path from the light source 21 and is at a position for receiving light reflected by the toothpaste-saliva foam 23. As explained earlier, there are no light transmission phenomena that need to be taken into account when investigating and analyzing toothpaste-saliva foam in the shown configuration.

In general, the invention, defined by the appended claims, covers the possibility that a power toothbrush or power flosser has an optical window at an appropriate position on the device, such as on a functional head of the device or on a neck portion or body portion, and a light-emitting unit and a light-receiving unit associated with the optical window. It is practical for the optical window to be arranged at a position of an area where saliva accumulates during use of the oral care appliance, which may be the position of a recess, as explained.

It is possible to keep track of a position of (a part of) the power toothbrush or power flosser with respect to the mouth. For example, if a toothbrush is used, information about the actual position of the brush head 71 may continually be available on the basis of suitable control and/or detection measures. In the context of the invention, such information may be used for obtaining gum health indications at a local level, so that areas of reduced/poor gum health in the mouth may be distinguished from areas of acceptable/good gum health. In this respect, it is noted that it may be advantageous to monitor time dependence of the light reflection during an oral care action, assuming that when a notable decrease of the reflection signal is found, this is caused by (additional) optical absorption following from release of blood. Thus, by monitoring time dependence of the signal that is obtained from the oral care appliance, it is possible to keep track of moments at which a release of blood is invoked. In combination with positioning information, this may help in finding "hot spots" in the mouth, i.e. areas which are made to bleed easily and which may be assumed to have poor gum health.

In the context of power toothbrushes or power flossers, it is possible that a cradle of the power toothbrush or power flosser is provided, and that an analysis unit 26 and an associated output interface are incorporated in the cradle. In examples that do not fall within the scope of the appended claims, an oral care appliance does not necessarily need to have an optical window and an associated light source 21 and light detector 25 arranged in a part of the device that is destined to be put into a person's mouth during use of the device. The fact is that it is also possible that an oral care appliance is equipped with components as mentioned at a bottom of a handle portion thereof, in which case the device is suitable to be used for detecting light reflection characteristics of a volume of saliva that has been spit on a surface.

Designing a power toothbrush or power flosser for putting the invention to practice involves the advantage that the detection of blood traces for the purpose of assessing a person's gum health can be performed automatically during an oral care action, or requires only minimum additional effort without the need for additional tools.

It will be clear to a person skilled in the art that the scope of the invention is not limited to the examples discussed in the foregoing, but that several amendments and modifications thereof are possible without deviating from the scope of the invention as defined in the attached claims. It is intended that the invention be construed as including all such amendments and modifications insofar they come within the scope of the claims. While the invention has been illustrated and described in detail in the figures and the description, such illustration and description are to be considered illustrative or exemplary only, and not restrictive. The invention is not limited to the disclosed embodiments. The drawings are schematic, wherein details which are not required for understanding the invention may have been omitted, and not necessarily to scale.

Variations to the disclosed embodiments can be understood and effected by a person skilled in the art in practicing the claimed invention, from a study of the figures, the description and the attached claims. In the claims, the word "comprising" does not exclude other steps or elements, and the indefinite article "a" or "an" does not exclude a plurality. Any reference signs in the claims should not be construed as limiting the scope of the invention.

Elements and aspects discussed for or in relation with a particular embodiment may be suitably combined with elements and aspects of other embodiments, unless explicitly stated otherwise. Thus, the mere fact that certain measures are recited in mutually different dependent claims does not indicate that a combination of these measures cannot be used to advantage.

The term "comprise" as used in this text will be understood by a person skilled in the art as covering the term "consist of'. Hence, the term "comprise" may in respect of an embodiment mean "consist of', but may in another embodiment mean "contain/include at least the defined species and optionally one or more other species".

In the present text, denoting light absorption as higher or relatively high on the one hand and as lower or relatively low on the other hand implies that the one light absorption is notably higher than the other light absorption. With reference to an absorption spectrum, higher or relatively high light absorption is related to peaks of the spectrum, whereas lower or relatively low light absorption is not and is rather related to valleys of the spectrum.

The invention involves an optical analysis of saliva or a fluid-saliva mixture in order to check whether the saliva or fluid-saliva mixture contains blood, which allows for determining whether or not a person may suffer from gingivitis or another condition affecting gum health. In particular, light received from a representative volume of fluid 23 containing saliva is detected and analyzed. The analysis involves determination of at least one measurement value of light received by a light-receiving unit 25 for only single wavelength of the light, particularly a wavelength that is associated with high absorption by a constituent of blood. It this respect, it is practical if the light-receiving unit 25 is configured to receive reflected light back from the volume of fluid 23. The optical analysis may be performed real-time during an action in a person's mouth involving a gum agitation effect, or after such action has taken place, for example.

## Claims

1. System configured to provide an indication of a person's gum health on the basis of an evaluation of the presence of blood traces in a volume of fluid (23) containing the person's saliva, the system comprising:
- an oral care appliance configured to perform brushing or flossing, wherein the oral care appliance is a power toothbrush having a brush head (71) and bristle tufts (72) or a power flosser;
- a light-emitting unit (21) configured to emit light to a volume of fluid (23) containing the person's saliva obtained during brushing or flossing using the oral care appliance, wherein the volume of fluid is at an area at which saliva accumulates on a window of the oral care appliance,
- a light-receiving unit (25) configured to receive light back from the volume of fluid (23), wherein at least the light-emitting unit and the light-receiving unit are incorporated in the oral care appliance and associated with the optical window, and
- an analysis unit (26) configured to perform an analysis of light received by the light-receiving unit (25) and to provide output representative of the person's gum health, and configured to execute at least one algorithm that is designed to determine at least one measurement value of light received by the light-receiving unit (25) for no more than a single wavelength of the light related to relatively high light absorption by a constituent of blood.

2. System according to claim 1, wherein the light-receiving unit (25) is configured to receive reflected light back from the volume of fluid (23).

3. System according to claim 1 or 2, wherein the at least one algorithm is designed
- to determine measurement values real-time during an action in the person's mouth involving a gum agitation effect, and
- to monitor a development of the measurement values over time and assess whether a deviation from a gradual course of the development of the measurement values occurs in the development.

4. System according to claim 3, wherein the at least one algorithm is designed
- to determine the measurement values at successive discrete moments in time, and
- to involve determining differences between successive measurement values in assessing whether a deviation from a gradual course of the development of the measurement values occurs in the development.

5. System according to claim 3 or 4, wherein the at least one algorithm is designed
- to determine that the measurement values are indicative of the presence of blood in a volume of fluid (23) containing saliva when a deviation from a gradual course of the development of the measurement values is found in the development.

6. System according to any of claims 1-5, wherein the light-emitting unit (21) is configured to emit light at only the single wavelength and/or the light-receiving unit (25) comprises a bandpass filter/light detector combination including a bandpass filter that is configured to only allow light at the single wavelength or a narrow wavelength band to pass.

7. System according to any of claims 1-6, comprising a remote device having a processor, wherein the analysis unit (26) comprises the processor of the remote device, and wherein the at least one algorithm to be executed by the analysis unit (26) is defined by an app installed on the remote device.

8. Method of providing an indication of a person's gum health on the basis of an evaluation of the presence of blood traces in a volume of fluid (23) containing the person's saliva, comprising:
- receiving a volume of fluid (23) containing the person's saliva during brushing or flossing using an oral care appliance, wherein the oral care appliance is a power toothbrush having a brush head (71) and bristle tufts (72) or a power flosser, wherein the volume of fluid is at an area at which saliva accumulates on a window of the oral care appliance,
- emitting light to the volume of fluid (23) containing the person's saliva and receiving the light back from the volume of fluid (23) using a light-emitting unit and a light-receiving unit which are incorporated in the oral care appliance and associated with the optical window, and analyzing the received light, and
- determining at least one measurement value of the light received back from the volume of fluid (23) for no more than a single wavelength of the light related to relatively high light absorption by a constituent of blood.

9. Method according to claim 8, comprising monitoring a development of measurement values over time and assessing whether a deviation from a gradual course of the development of the measurement values occurs in the development.

10. Method according to claim 9, comprising determining that the measurement values are indicative of the presence of blood in a volume of fluid (23) containing saliva when a deviation from a gradual course of the development of the measurement values is found in the development.

11. Computer program product comprising code to cause a processor, when the code is executed on the processor, to cause the system according to any one of claims 1 to 7 to execute the method according to any of claims 8-10.

12. Remote device having a processor, comprising the computer program product according to claim 11.

## Patentansprüche

1. System, das dazu konfiguriert ist, einen Hinweis auf die Zahnfleischgesundheit einer Person auf der Grundlage einer Auswertung des Vorhandenseins von Blutspuren in einem Flüssigkeitsvolumen (23), das den Speichel der Person enthält, bereitzustellen, das System umfassend:
- ein Mundpflegegerät, das dazu konfiguriert ist, Zähneputzen oder Reinigen mit Zahnseide durchzuführen, wobei das Mundpflegegerät eine elektrische Zahnbürste, die einen Bürstenkopf (71) und Borstenbüscheln (72) aufweist, oder eine elektrische Zahnseideapparatur ist;
- eine Licht emittierende Einheit (21), die dazu konfiguriert ist, Licht an ein Flüssigkeitsvolumen (23) zu emittieren, das den Speichel der Person enthält, der während des Zähneputzens oder des Reinigens mit Zahnseide unter Verwendung des Mundpflegegeräts gewonnen wurde, wobei sich das Flüssigkeitsvolumen in einem Bereich befindet, in dem sich Speichel auf einem Fenster des Mundpflegegeräts ansammelt,
- eine Licht empfangende Einheit (25), die dazu konfiguriert ist, Licht von dem Flüssigkeitsvolumen (23) zurück zu empfangen, wobei mindestens die Licht emittierende Einheit und die Licht empfangende Einheit in das Mundpflegegerät eingebunden und zu dem optischen Fenster zugehörig sind, und
- eine Analyseeinheit (26), die dazu konfiguriert ist, eine Analyse des von der Licht empfangenden Einheit (25) empfangenen Lichts durchzuführen und eine Ausgabe bereitzustellen, die für die Zahnfleischgesundheit der Person repräsentativ ist, und die dazu konfiguriert ist, mindestens einen Algorithmus auszuführen, der dazu ausgelegt ist, mindestens einen Messwert des von der Licht empfangenden Einheit (25) empfangenen Lichts für nicht mehr als eine einzige Wellenlänge des Lichts zu bestimmen, die mit einer relativ hohen Lichtabsorption durch einen Bestandteil des Bluts im Zusammenhang steht.

2. System nach Anspruch 1, wobei die Licht empfangende Einheit (25) dazu konfiguriert ist, vom Flüssigkeitsvolumen (23) reflektiertes Licht zurück zu empfangen.

3. System nach Anspruch 1 oder 2, wobei der mindestens eine Algorithmus dazu ausgelegt ist,
- Messwerte während einer Handlung im Mund der Person, die eine Zahnfleischreizwirkung involviert, in Echtzeit zu bestimmen, und
- eine Entwicklung der Messwerte mit der Zeit zu beobachten und zu beurteilen, ob bei der Entwicklung eine Abweichung von einem allmählichen Verlauf der Entwicklung der Messwerte auftritt.

4. System nach Anspruch 3, wobei der mindestens eine Algorithmus dazu ausgelegt ist,
- die Messwerte zu aufeinanderfolgenden diskreten Zeitpunkten zu bestimmen, und
- ein Bestimmen von Unterschieden zwischen aufeinanderfolgenden Messwerten beim Beurteilen, ob bei der Entwicklung eine Abweichung von einem allmählichen Verlauf der Entwicklung der Messwerte auftritt, zu involvieren.

5. System nach Anspruch 3 oder 4, wobei der mindestens eine Algorithmus dazu ausgelegt ist,
- zu bestimmen, dass die Messwerte auf das Vorhandensein von Blut in einem Speichel enthaltenden Flüssigkeitsvolumen (23) hinweisen, wenn bei der Entwicklung eine Abweichung von einem allmählichen Verlauf der Entwicklung der Messwerte festgestellt wird.

6. System nach einem der Ansprüche 1-5, wobei die Licht emittierende Einheit (21) dazu konfiguriert ist, Licht nur bei der einzigen Wellenlänge zu emittieren und/oder die Licht empfangende Einheit (25) eine Bandpassfilter/Lichtdetektor- Kombination umfasst, die einen Bandpassfilter einschließt, der dazu konfiguriert ist, Licht nur bei einer einzigen Wellenlänge oder einem schmalen Wellenlängenband durchzulassen.

7. System nach einem der Ansprüche 1-6, umfassend eine entfernte Vorrichtung, die einen Prozessor aufweist, wobei die Analyseeinheit (26) den Prozessor der entfernten Vorrichtung umfasst, und wobei der mindestens eine von der Analyseeinheit (26) auszuführende Algorithmus durch eine auf der entfernten Vorrichtung installierten Anwendung definiert ist.

8. Verfahren zum Bereitstellen eines Hinweises auf die Zahnfleischgesundheit einer Person auf der Grundlage einer Auswertung des Vorhandenseins von Blutspuren in einem Flüssigkeitsvolumen (23), das den Speichel der Person enthält, umfassend:
- Empfangen eines Flüssigkeitsvolumens (23), das den Speichel der Person enthält, während des Zähneputzens oder des Reinigens mit Zahnseide unter Verwendung eines Mundpflegegeräts, wobei das Mundpflegegerät eine elektrische Zahnbürste, die einen Bürstenkopf (71) und Borstenbüschel (72) aufweist, oder eine elektrische Zahnseideapparatur ist, wobei sich das Flüssigkeitsvolumen in einem Bereich befindet, in dem sich Speichel auf einem Fenster des Mundpflegegeräts ansammelt,
- Emittieren von Licht an das Flüssigkeitsvolumen (23), das den Speichel der Person enthält, und Empfangen des Lichts zurück von dem Flüssigkeitsvolumen (23) unter Verwendung einer Licht emittierenden Einheit und einer Licht empfangenden Einheit, die in das Mundpflegegerät eingebunden und zu dem optischen Fenster zugehörig sind, und Analysieren des empfangenen Lichts, und
- Bestimmen von mindestens einem Messwert des von dem Flüssigkeitsvolumen (23) zurück empfangenen Lichts für nicht mehr als eine einzige Wellenlänge des Lichts, die mit einer relativ hohen Lichtabsorption durch einen Bestandteil des Bluts im Zusammenhang steht.

9. Verfahren nach Anspruch 8, umfassend ein Beobachten einer Entwicklung von Messwerten über die Zeit und ein Bewerten, ob bei der Entwicklung eine Abweichung von einem allmählichen Verlauf der Entwicklung der Messwerte auftritt.

10. Verfahren nach Anspruch 9, umfassend ein Bestimmen, dass die Messwerte auf das Vorhandensein von Blut in einem Speichel enthaltenden Flüssigkeitsvolumen (23) hinweisen, wenn bei der Entwicklung eine Abweichung von einem allmählichen Verlauf der Entwicklung der Messwerte festgestellt wird.

11. Computerprogrammprodukt, umfassend Code, um einen Prozessor zu veranlassen, wenn der Code auf dem Prozessor ausgeführt wird, das System nach einem der Ansprüche 1 bis 7 zu veranlassen, das Verfahren nach einem der Ansprüche 8-10 auszuführen.

12. Entfernte Vorrichtung, die einen Prozessor aufweist, umfassend das Computerprogrammprodukt nach Anspruch 11.

## Revendications

1. Système configuré pour fournir une indication sur l'état de santé de la gencive d'une personne sur la base d'une évaluation de la présence ou non de traces de sang dans un volume de fluide (23) contenant de la salive de la personne, le système comprenant :
- un appareil de soins bucco-dentaires configuré pour effectuer un brossage ou un passage de fil dentaire, dans lequel l'appareil de soins bucco-dentaires est une brosse à dents électrique présentant une tête de brosse (71) et des touffes de poils (72) ou un fil dentaire électrique ;
- une unité électroluminescente (21) configurée pour émettre de la lumière vers un volume de fluide (23) contenant de la salive de la personne obtenue lors du brossage ou du passage du fil dentaire à l'aide de l'appareil de soins bucco-dentaires, dans lequel le volume de fluide se trouve au niveau d'une zone au niveau de laquelle de la salive s'accumule sur une fenêtre de l'appareil de soins bucco-dentaires,
- une unité de réception de lumière (25) configurée pour recevoir la lumière en retour du volume de fluide (23), dans lequel au moins l'unité électroluminescente et l'unité de réception de lumière sont incorporées dans l'appareil de soins bucco-dentaires et associées à la fenêtre optique, et
- une unité d'analyse (26) configurée pour effectuer une analyse de la lumière reçue par l'unité de réception de lumière (25) et pour fournir une sortie représentative de l'état de santé de la gencive de la personne, et configurée pour exécuter au moins un algorithme conçu pour déterminer au moins une valeur de mesure de la lumière reçue par l'unité de réception de lumière (25) pour pas plus d'une seule longueur d'onde de la lumière liée à une absorption de lumière relativement élevée par un constituant du sang.

2. Système selon la revendication 1, dans lequel l'unité de réception de lumière (25) est configurée pour recevoir la lumière réfléchie en retour du volume de fluide (23).

3. Système selon la revendication 1 ou 2, dans lequel le au moins un algorithme est conçu
- pour déterminer des valeurs de mesure en temps réel pendant une action dans la bouche de la personne impliquant un effet d'agitation de la gencive, et
- pour suivre un développement des valeurs de mesure au fil du temps et évaluer si un écart par rapport à une évolution progressive des valeurs de mesure se produit au cours du développement.

4. Système selon la revendication 3, dans lequel le au moins un algorithme est conçu
- pour déterminer les valeurs de mesure à des instants distincts successifs, et
- pour impliquer la détermination de différences entre des valeurs de mesure successives de manière à évaluer si un écart par rapport à une évolution progressive du développement des valeurs de mesure se produit au cours du développement.

5. Système selon la revendication 3 ou 4, dans lequel le au moins un algorithme est conçu
- pour déterminer si les valeurs de mesure indiquent la présence de sang dans un volume de fluide (23) contenant de la salive lorsqu'un écart par rapport à une évolution progressive du développement des valeurs de mesure est constaté lors du développement.

6. Système selon l'une quelconque des revendications 1-5, dans lequel l'unité électroluminescente (21) est configurée pour émettre de la lumière uniquement au niveau de la seule longueur d'onde et/ou l'unité de réception de lumière (25) comprend une combinaison filtre passe-bande/détecteur de lumière incluant un filtre passe-bande qui est configuré pour laisser passer uniquement la lumière au niveau de la seule longueur d'onde ou d'une bande de longueur d'onde étroite.

7. Système selon l'une quelconque des revendications 1-6, comprenant un dispositif à distance présentant un processeur, dans lequel l'unité d'analyse (26) comprend le processeur du dispositif à distance et dans lequel le au moins un algorithme qui doit être exécuté par l'unité d'analyse (26) est défini par une application installée sur le dispositif à distance.

8. Procédé de fourniture d'une indication sur l'état de santé de la gencive d'une personne sur la base d'une évaluation de la présence ou non de traces de sang dans le volume de fluide (23) contenant de la salive de la personne, comprenant :
- la réception d'un volume de fluide (23) contenant de la salive de la personne pendant le brossage ou le passage du fil dentaire à l'aide d'un appareil de soins bucco-dentaires, dans lequel l'appareil de soins bucco-dentaires est une brosse à dents électrique présentant une tête de brosse (71) et des touffes de poils (72) ou un fil dentaire électrique, dans lequel le volume de fluide se trouve au niveau d'une zone au niveau de laquelle de la salive s'accumule sur une fenêtre de l'appareil de soins bucco-dentaires,
- l'émission de lumière vers le volume de fluide (23) contenant de la salive de la personne et la réception de lumière en retour du volume de fluide (23) à l'aide d'une unité électroluminescente et d'une unité de réception de lumière qui sont incorporées dans l'appareil de soins bucco-dentaires et associées à la fenêtre optique, et l'analyse la lumière reçue, et
- la détermination d'au moins une valeur de mesure de la lumière reçue en retour du volume de fluide (23) pour pas plus d'une seule longueur d'onde de la lumière liée à une absorption de lumière relativement élevée par un constituant du sang.

9. Procédé selon la revendication 8, comprenant le suivi d'un développement de valeurs de mesure au fil du temps et le fait d'évaluer si un écart par rapport à une évolution progressive du développement des valeurs de mesure se produit au cours du développement.

10. Procédé selon la revendication 9, comprenant la détermination que les valeurs de mesure indiquent la présence de sang dans un volume de fluide (23) contenant de la salive lorsqu'un écart par rapport à une évolution progressive du développement des valeurs de mesure est constaté au cours du développement.

11. Produit de programme informatique comprenant un code pour amener un processeur, lorsque le code est exécuté sur le processeur, à amener le système selon l'une quelconque des revendications 1 à 7 à exécuter le procédé selon l'une quelconque des revendications 8 à 10.

12. Dispositif à distance présentant un processeur, comprenant le produit de programme informatique selon la revendication 11.
